# EUROPÄISCHE PATENTSCHRIFT

(11) **EP 1 115 688 B1**
(45) Veröffentlichungstag und Bekanntmachung des Hinweises auf die Patenterteilung: **03.12.2003**
(21) Anmeldenummer: 99948761.4
(22) Anmeldetag: 16.09.1999
(51) Int. Cl.: C07C 67/333, C07C 69/54

(54) **VERFAHREN ZUR DEPOLYMERISATION VON POLYMETHYLMETHACRYLAT**
METHOD FOR DEPOLYMERISING POLYMETHYLMETHACRYLATE
PROCEDE DE POLYMERISATION DE POLYMETHYLMETHACRYLATE

(30) Priorität: 21.09.1998 DE 19843112
(43) Veröffentlichungstag der Anmeldung: 18.07.2001
(73) Patentinhaber: MG Technologies AG, 60325 Frankfurt am Main (DE); Röhm GmbH & Co. KG, 64293 Darmstadt (DE)
(72) Erfinder: WEISS, Hans-Jürgen, D-61440 Oberursel (DE); SCHMALFELD, Jörg, D-61352 Bad Homburg (DE); ZENTNER, Udo, D-64347 Griesheim (DE); GROSCHANG, Tobias, D-63456 Hanau (DE); GROPP, Udo, D-69469 Weinheim (DE); FUSS, Werner, D-63791 Karlstein (DE); GOEDECKE, Ralf, D-63517 Rodenbach (DE); SCHÖLA, Egbert, A-7052 Müllendorf (AT)
(86) Internationale Anmeldenummer: EP9906852
(87) Internationale Veröffentlichungsnummer: WO00017149

(56) Entgegenhaltungen:
- DE-A- 19 724 074
- FR-A- 809 386
- FR-A- 1 114 639
- US-A- 5 663 420

## Beschreibung

### Beschreibung

Die vorliegende Erfindung richtet sich auf ein Verfahren zur Rückgewinnung von monomeren Estern substituierter oder unsubstituierter Acrylsäuren aus entsprechende Struktureinheiten aufweisendem Polymermaterial.

Acrylat-Polymere, zu denen vorwiegend aus Polymethylmethacrylat (PMMA) bestehende Acrylgläser gehören, werden unter anderem zur Herstellung langlebiger Gebrauchsgüter verwendet. Hierzu werden häufig Formverfahren benützt, in deren Verlauf erhebliche Mengen an Abfallpolymer anfallen können. Zur sinnvollen Aufarbeitung dieser Produktionsabfälle sowie zur Verwertung von aus dem Gebrauchsprozess zurückgeführten Altstoffen hat es nun schon eine ganze Reihe von Vorschlägen gegeben.

Es ist die Tatsache bekannt, daß Acrylatpolymere, vor allem PMMA, zu den wenigen Kunststoffen zählen, die hervorragend für das direkte chemische Recycling geeignet sind. Hierunter ist zu verstehen, daß diese Polymere sich bei bestimmten Temperaturen und Drücken wieder vollständig in die entsprechenden Monomerbausteine zerlegen lassen (Depolymerisation), wenn auf geeignete Weise Wärme zugeführt wird (Grassie, N., Melville, H. W., Bull. Soc. Chim. Belges 1948, S. 142).

In den Berichten zum 19. Kunststofftechnischen Kolloquium des Eurogress Aachen (11.-13.3.1998) wird ein kontinuierlich arbeitendes Verfahren zur PMMA-Depolymerisation beschrieben. Dabei wird der Kunststoff zerkleinert in einen heißen Extruder (ZSK 30) gegeben, in dem zwei dichtkämmende Schnecken mit selbstreinigender Wirkung rotieren. Diese Schnecken fördern nicht depolymerisiertes PMMA und andere Rückstände aus dem Extruder heraus. Das PMMA depolymerisiert aufgrund der thermischen und der mechanischen Scherung im Extruder. Das entstehende MMA wird als Dampfphase über den Entgasungsdom abgezogen und kondensiert. Der MMA-Gehalt im Kondensat schwankt bei diesem Verfahren zwischen 89 % und 97 %, die Ausbeute an MMA liegt bei <97 %. Bei dem eben beschriebenen Verfahren erfolgt die Erwärmung des PMMA im Extruder über die Mantelwände. Das Verhältnis von Wandfläche zu Reaktorvolumen verschlechtert sich jedoch mit größerwerdender Anlage. Für Großanlagen im industriellen Maßstab ist die zur Verfügung stehende Mantelfläche derart gering, daß man den Extruder entweder immens heiß machen muß, um das PMMA in ausreichendem Maße zu zerlegen, oder man erhält nur sehr viel schlechtere Ausbeuten an MMA. Durch die notwendige verstärkte Aufheizung des Extrudermantels treten allerdings lokale Überhitzungen auf, die zur Nebenproduktbildung beitragen und die Monomerreinheit beeinträchtigen.

Weiterhin ist bekannt, PMMA mittels Wirbelschichtpyrolyse zu depolymerisieren. Als Wirbelgut wird Quarzsand der Körnung 0,3-0,7 mm benutzt. Nachteil an diesem Verfahren ist, daß das Wirbelgut mit der Zeit mit Ruß graphitiert wird. Der Ruß kann, wenn er vom Sandkorn abplatzt mit dem Gasstrom mitgerissen werden. Um ein ansprechend sauberes Monomer zu erhalten, ist deshalb bei dieser Anlage die Implementierung von vielen speziellen Filtersystemen Voraussetzung (Kühler, Zyklon, Elektrofilter). Zur Verwirbelung des Sandes wird bei diesem Verfahren ein Stickstoffstrom eingesetzt. Ebenfalls nachteilig ist, daß nach der Depolymerisation der Stickstoff und das MMA-Gas durch Kühlung wieder getrennt werden muß. Der Stickstoffstrom, der nach der Trennung vom Produktgas wieder dem Reaktor zugeführt wird, muß deshalb in ständigem Wechsel abgekühlt und aufgeheizt werden, wobei die Temperaturdifferenz mindestens 400 K beträgt. Für ein großtechnisches Verfahren ist dies aus ökonomischer und ökologischer Sicht nachteilig (J. Franck, Doktorarbeit 1993 Universität Hamburg).

In der EP-B1-0600727 bzw. US 5,663,420 wird ein Depolymerisationsverfahren beschrieben, bei dem z.B. PMMA in einem Fließbett mit heißen, verwirbelten, feinkörnigen, festen Wärmeträgern in Kontakt gebracht wird, die dabei entstehenden Dämpfe abgeleitet und kondensiert werden, der heiße Wärmeträger kontinuierlich an einem Ende des Reaktors zugeführt und am anderen Ende ausgetragen wird.

Weiterhin werden in der FR-A-1114639 und FR-A-809386 Verfahren zur Rückgewinnung von monomeren Estern durch Depolymerisation beschrieben.

Aufgabe der Erfindung ist es, ein Verfahren zur Rückgewinnung von monomeren Estern substituierter oder unsubstituierter Acrylsäuren aus entsprechende Struktureinheiten aufweisendem Polymermaterial anzugeben, welches eine kontinuierliche, rückstandsfreie Depolymerisation gestattet und so die Herstellung von qualitativ hochwertigen rezyklierten monomeren Estern in hoher Ausbeute erlaubt. Unter rückstandsfrei wird im Sinne der Erfindung ein Verfahren angesehen, welches im Reaktorraum die Ausbildung von Ablagerungen vermeidet und somit das Abschalten der Anlage zur Entfernung der Ablagerungen überflüssig macht, womit ein kontinuierlicher Betrieb gewährleistet wird.

Aufgabe der Erfindung ist weiterhin die Angabe eines wie oben genannten Verfahrens, welches im industriellen Maßstab betrieben werden kann und die Nachteile wie schlechter Wärmeübergang bei der Depolymerisation, hoher apparativer Aufwand sowie energetisch ungünstige Prozeßflüsse vermeiden hilft.

Gegenstand der Erfindung ist ein Verfahren zur Depolymerisation von PMMA, welches dadurch gekennzeichnet ist, daß man das Polymermaterial in einem Reaktor mit heißem mechanisch verwirbelten Feststoff (Wärmeträger) in Kontakt bringt und man die dabei entstehenden Dämpfe ableitet und kondensiert, wobei man den heißen Wärmeträger kontinuierlich an einem Ende des Reaktors zuführt und am anderen Ende abgekühlten Wärmeträger austrägt.

Aufgrund des erfindungsgemäßen Verfahrens gelingt es durch die sehr gute Wärmeübertragung des feinkörnigen Feststoffs und der damit verbundenen relativ kurzen Verweilzeit des Polymermaterials, das Reaktorvolumen klein zu halten. Daraus ergibt sich auch eine Verweildauer der entstehenden Monomerdämpfe im Reaktor von weniger als 6 Sekunden. Man erhält die gewünschten Ester der Acrylsäuren in sehr guten Ausbeuten und mit hoher Reinheit. Mithin sorgt der heiße feinkörnige Wärmeträger auch dafür, daß bei Großanlagen ein ausreichender Wärmeübergang bei der Depolymerisation gewährleistet ist.

Durch den mechanisch verwirbelten feinkörnigen Wärmeträger stellt sich erfindungsgemäß im Reaktor ein Reibeeffekt ein, der ein Anbacken von aus der Depolymerisation stammenden Nebenprodukten an den Reaktorinnenwänden und - inneneinrichtungen gänzlich unterbinden hilft. Diese Nebenprodukte der Depolymerisation werden mit dem feinkörnigen Wärmeträger aus dem Reaktor kontinuierlich ausgetragen, wodurch eine Anhäufung der Nebenprodukte im Reaktor verhindert wird. Es ist somit möglich, das vorteilhafte Verfahren kontinuierlich durchzuführen, da keine Rückstände der Depolymerisation, welche ansonsten von Zeit zu Zeit aus entsprechenden Anlagen des Standes der Technik entfernt werden müssen, im Reaktor zurückbleiben. Der Monomergasstrom, welcher den Reaktor verläßt, ist ausreichend rein und braucht nur durch einen Zyklon von mitgerissenen Staubpartikeln befreit zu werden. Auf keinen Fall ist eine Trennung von einem Trägergasstrom, der auch mehr Staubpartikel mitreißen würde, notwendig.

Die mechanische Verwirbelung und den Transport des feinkörnigen Wärmeträgers kann man dabei durch alle dem Fachmann geläufigen Möglichkeiten, wie z. B. durch bewegte oder rotierende Wände ggf. unter Einfluß der Gravitation, bewerkstelligen. Bevorzugt ist die Ausführungsform, bei der man die dem Reaktor zugeführten Substanzen in einem Mischwerk durch eine oder mehrere ineinandergreifende, gleichsinnig rotierende Wellen, die mit Wendeln oder anderen Mischwerkzeugen versehen sind, mechanisch verwirbelt, miteinander vermischt und fördert. Hierbei kann eine annähernd gleiche Verweilzeit aller festen Partikel (Pfropfenströmung) im Bereich von 5-60 Sekunden durch Veränderung der Schneckendrehzahl eingestellt werden.

Dabei wird das Polymermaterial von den durch die Wendeln oder Mischwerkzeuge verwirbelten, heißen Wärmeträger in kurzer Zeit aufgeheizt und depolymerisiert. Die flüchtigen Bestandteile werden abgeleitet, wohingegen die nach der Depolymerisation zurückbleibenden festen Nebenprodukte mit dem Wärmeträger aus dem Reaktor herausgetragen werden, wodurch ebenfalls sehr vorteilhaft einer Kontamination der abgeleiteten Monomerdämpfe mit aus den festen Nebenprodukten stammenden Bestandteilen entgegengewirkt wird. Die Massenbilanz des Wärmeträgers im Reaktor wird bevorzugt durch Supplementierung am Kopfende aus einer erhitzten Vorlage aufrechterhalten.

Der Transport des Wärmeträgers im Reaktor kann, wie oben angedeutet, bevorzugt durch eine oder mehrere rotierende Wellen, die mit Wendeln oder anderen Mischwerkzeugen ausgestattet sind, von der Eintragsöffnung zur Austragsöffnung bewerkstelligt werden.

Der Wärmeträger kann nach dem Austrag aus dem Reaktor über einen Nachentgasungsbehälter zum Fuß einer pneumatischen Förderstrecke geführt werden. Die folgende Aufheizung des Wärmeträgers kann prinzipiell mit allen dem Fachmann geläufigen Methoden erfolgen. Bevorzugt werden zum Fuß der pneumatischen Förderstrecke über die Brennkammer ein heißer, ggf. sauerstoffhaltiger Gasstrom und/oder evtl. Zusatzbrennstoffe zugeführt. Der resultierende Gasstrom fördert den Wärmeträger nach oben, wobei gleichzeitig brennbare Rückstände aus der Depolymerisation und Zusatzbrennstoffe verbrannt werden. Dies führt mithin zur Rückerhitzung des feinkörnigen Wärmeträgers. Das Gemisch aus Wärmeträger und Gasen gelangt in einen Wärmeträgerabscheider, aus dem Gase und feine Staubpartikel (z. B. im PMMA enthaltene Farbpigmente) über einen Staubabscheider (Zyklon, Abgasfilter) abgezogen werden. Der im Wärmeträgerabscheider separierte Wärmeträger rieselt nach unten und gelangt in einen Sammelbehälter, der als heiße Wärmeträger-Vorlage für den Reaktor dient.

Die Temperatur des Wärmeträger-Feststoffes am Reaktoreintritt hängt vom Verhältnis der Massenströme Wärmeträger/Polymermaterial ab. Bei einem Verhältnis von 10:1 ergibt sich eine Überhitzung des Wärmeträgers von 150 °C, bei einem Verhältnis von 5 : 1 eine von 300 °C. Die Reaktions-Mischtemperatur des Wärmeträgers kann bei der Depolymerisation zwischen 300 °C und 650 °C, bevorzugt zwischen 350 °C bis 450 °C, liegen. Der durch den Heißgasstrom aufgewärmte Wärmeträger hat hingegen eine Temperatur von 400 °C bis 900 °C, bevorzugt sind 500 °C bis 750 °C.

Als Wärmeträger kann jeder anorganische feinkörnige Feststoff (Korngröße zwischen 0,1 und 5 mm, bevorzugt 0,3 und 2,0 mm) verwendet werden, der über die erforderliche Festigkeit verfügt und eine ausreichende Beständigkeit gegenüber Temperaturwechseln und Sauerstoff aufweist. In vielen Fällen hat sich gesiebter Sand bewährt, der nach DIN 4222 als Grobsand bezeichnet wird. Geeignet sind aber auch andere natürlich vorkommende oder synthetisch erzeugte Oxide auf der Basis von Silizium, Aluminium, Magnesium, Zirkonium oder auch Mischungen aus diesen Elementen.

Die Verweilzeit der im Reaktor gebildeten Dämpfe und Gase vor dem Kondensator kann mit dem erfindungsgemäßen Verfahren weniger als 6 Sekunden, bevorzugt weniger als 2 Sekunden, betragen. Die Verweilzeit des feinkörnigen Wärmeträgers im Reaktor ist frei wählbar. Vorzugsweise liegt sie im Bereich von 5 bis 60 Sekunden.

Das Verhältnis von heißem Wärmeträger zu PMMA im Reaktor ist ebenfalls in weiten Bereichen frei wählbar. Sinnvoll und bevorzugt erscheint ein Verhältnis zwischen 3 : 1 und 30 : 1.

In einem weiteren Aspekt kann das erfindungsgemäße Verfahren die Wirtschaftlichkeit der Rückgewinnung sowie die Qualität des erhaltenen Produkts dadurch verbessern, daß man die abgeleiteten Dämpfe mit Kondensat, welches in einem Monomer-Kreislaufkühler abgekühlt wurde, behandelt und daß man das beim Behandeln resultierende Kondensat in den Kühler zurückleitet, wo man es abkühlt und teilweise zum Behandeln der abgeleiteten Dämpfe rückführt, und man den übrigen Kondensat-Teil zur weiteren Aufarbeitung und Produktgewinnung ausschleust.

Hierbei wird das rohe dampfförmige Depolymerisat, also die Depolymerisationsgase, zunächst mit einem Teil des bereits vorher in einem Monomer-Kreislaufkühler abgekühlten und rückgeführten Kondensat mittels einer Düse wie in einer Dusche besprüht. Durch den Direktkontakt der Depolymerisationsgase mit der verdüsten Roh-Kondensat-Flüssigkeit wird eine schnelle Intensivkühlung und eine sehr kurze Verweilzeit des dampfförmigen Depolymerisats bei Depolymerisationstemperatur erzielt, was zu einer deutlichen Verbesserung der Monomer-Ausbeute und Qualität führt. Die abgeleiteten Dämpfe werden hierbei im Gleichstromverfahren mit Kondensat gequencht. Hierdurch können Feststoffablagerungen, die auf den sonst üblichen Kühlern entstehen, deutlich reduziert werden. Da die eigentlichen Kühler nur mit einem deutlich kühleren, bereits kondensierten Rohprodukt in Kontakt kommen, ist die Gefahr des Anbackens oder der Ablagerung geringer. Eine Reduzierung der Belagdicke an Kühlern jedoch kann zusätzlich zu einer verbesserten Qualität des Rohkondensats, insbesondere zu einem höheren Monomergehalt, beitragen.

Das zum Behandeln der Monomergase verwendete Kondensat kann im Monomer-Kreislaufkühler auf eine Temperatur von etwa zwischen 5 und 40 °C, bevorzugt 20 und 30 °C, abgekühlt werden. Mit Teilen dieses abgekühlten Kondensats werden dann die aus dem Reaktor abgeleiteten Dämpfe beim Quenchen zweckmäßig auf eine Kondensat-Temperatur zwischen 20 und 50 °C, bevorzugt 35 und 50 °C, abgekühlt.

Die nicht kondensierbaren Dämpfe und Gase des Kondensats werden zweckmäßigerweise in die Steigleitung, in der der feinkörnige Wärmeträger aufgeheizt wird, eingeleitet und mitverbrannt.

Grundsätzlich kann das Polymermaterial in jeder denkbaren Form zum Reaktor gebracht werden. Bewährt haben sich die marktgängigen Eintragvorrichtungen in Verbindung mit Förderbändern, Schnecken oder ähnlichem. Größere Stücke wie Platten oder Formkörper lassen sich leicht auf die für den Reaktor erforderliche oder gewünschte Größe zerkleinern, beispielsweise durch vorgeschaltete Schredder oder Mühlen. Die Größe der verarbeitbaren Polymermaterialstücke richtet sich letztlich auch nach den Eigenschaften der Polymerisate und der Kapazität des Reaktors.

Allgemein kann das in den Reaktor einzuspeisende und dort zu depolymerisierende Polymermaterial in irgendeiner bequem verarbeitbaren Form vorliegen, wie beispielsweise als Schnitzel, als Granulat, fein als Pulver, als Späne, oder als grob geschreddertes Material. Diese Zugabeformen können entweder allein oder auch in Kombination eingespeist werden. Außerdem können die mehr oder weniger festen Zugabeformen bei der Einspeisung auch mit flüssigem Monomer zusammen, in mehr oder weniger reiner oder verunreinigter Form, eingespeist werden. Besonders bewährt haben sich bei Ausführung der Erfindung Granulate mit einer bevorzugten Korngröße von ca. 1 bis 10 mm.

Die im erfindungsgemäßen Verfahren in den Reaktor einzuspeisenden Polymere enthalten überwiegend Struktureinheiten, die hinsichtlich ihres chemischen Aufbaus der nachfolgenden Formel I gehorchen: 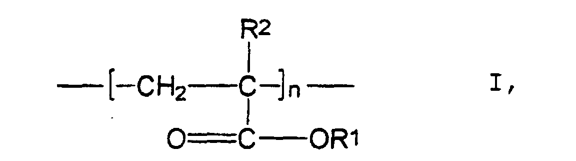 worin
R¹ C₁₋₆-Alkyl, bevorzugt C₁₋₄-Alkyl,
R² H, C₁₋₆-Alkyl, bevorzugt H oder C₁₋₄-Alkyl, ganz besonders bevorzugt H oder CH₃, und
n eine positive ganze Zahl größer 1
sind.

Zu beispielhaften Verbindungen gehören Polymethylacrylat, Polyethylacrylat, Polymethylmethacrylat, Polypropylacrylat, Polybutylacrylat, Polypropylmethacrylat, Polybutylmethacrylat und Copolymere, die zwei oder mehrere dieser Polymersorten aufweisen. Die ersten vier Verbindungen sind im Rahmen der Erfindung bevorzugt. Ganz besonders bevorzugt ist Polymethylmethacrylat (PMMA).

Neben den chemischen Mischungen (statistische Copolymere oder auch Blockcopolymere), die durch Copolymerisation von wenigstens zwei substituierten oder unsubstituierten Acrylsäureestermonomeren entstanden sind (z. B. Methylmethacrylat-n-Butylmethacrylat-Copolymere), lassen sich mit dem erfindungsgemäßen Verfahren auch Copolymere verarbeiten, die bis zu 50 Gew.-% wenigstens eines weiteren mit wenigstens einem substituierten oder unsubstituierten Acrylsäureestermonomeren copolymerisierbaren vinylisch ungesättigten Monomeren aufweisen. Beispiele hierfür sind u. a. Methylmethacrylat-Styrol-Copolymere oder Methylmethacrylat-Butylacrylat-Styrol-Terpolymere.

Auch physikalische Mischungen, sogenannte Blends, sind erfindungsgemäß wiederaufarbeitbar. Allgemein sind hinsichtlich der Wiederaufarbeitung von Polymermaterial nach dem erfindungsgemäßen Verfahren lediglich die grundsätzliche Möglichkeit der zerstörungsfreien Depolymerisation (in Bezug auf die Monomeren) sowie die Möglichkeit zur Trennung des entstehenden Dampfgemisches in einer fraktionierten Destillation oder mit anderen geläufigen Trennmethoden als limitierende Faktoren zu sehen. Sind Depolymerisation und Trennung prinzipiell möglich, besteht für die Anwendung des erfindungsgemäßen Verfahrens kein grundsätzliches Hindernis mehr.

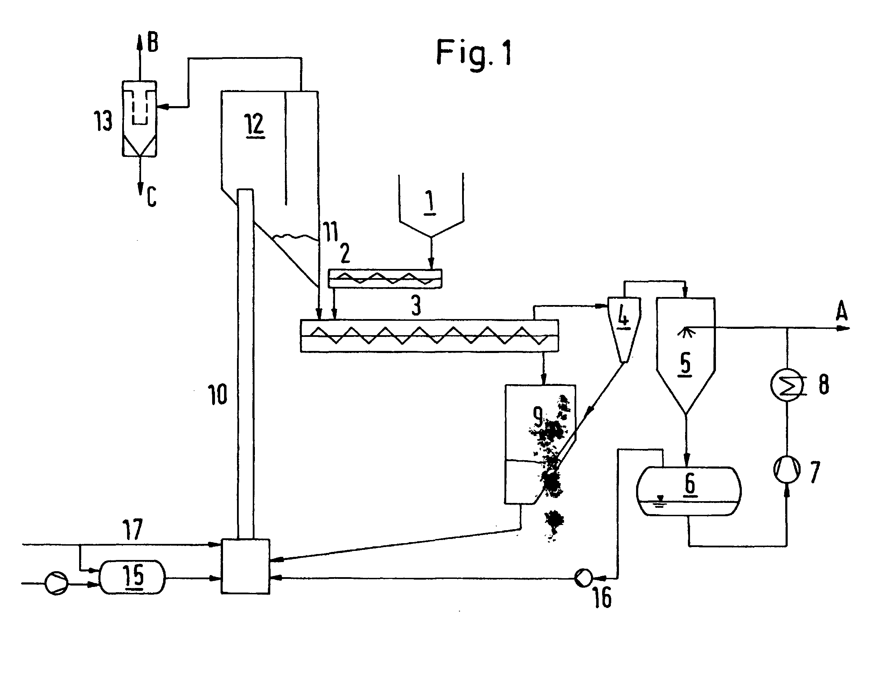

Nachfolgend wird die Erfindung unter Bezugnahme auf die Zeichnung in Figur 1 näher erläutert.

Figur 1 zeigt einen Vorlagebehälter 1 für das Polymermaterial, welches kopfseitig in eine Dosierschnecke 2 aufgegeben wird. Endseitig mündet die Dosierschnecke 2 in das kopfseitige Ende des Reaktors 3, welcher an diesem Ende zugleich eine Zuführeinrichtung für heißen Wärmeträger aus der Vorlage 11 besitzt. Der Reaktor 3 ist endständig zum einen mit einem Vorlagebehälter 9 (Gaser) für den ausgetragenen Wärmeträger verbunden, zum anderen mit einem Zyklon 4. Vom Zyklon 4 mündet eine weitere Zuführung in den Kondensator 5, dieser ist mit einem Monomerbehälter 6 verbunden. Dem Monomerbehälter 6 nachgeschaltet ist eine Monomerpumpe 7, welche über einen Monomer-Kreislaufkühler 8 zum einen den Kondensator 5 bedient, zum anderen Produkt A zum Austrag bereitstellt.

Der im Zyklon 4 abgeschiedene Staub wird in den Gaser 9 zurückgeführt, der wiederum eine Zuführung zur Steigleitung 10 hat. Aus dem Monomerbehälter 6 werden nicht kondensierbare Gase über ein Gebläse 16 ebenfalls zu der Steigleitung 10 gefördert, wo sie thermisch entsorgt (verbrannt) werden.

In die Steigleitung 10 wird weiterhin heißes, sauerstoffhaltiges Rauchgas aus der Brennkammer 15 und Brennstoff aus Leitung 17 aufgegeben. Die Brennkammer 15 wird ebenfalls mit Brennstoff aus Leitung 17 und Luft aus Gebläse 14 versorgt. Die Steigleitung 10 mündet oben in den Wärmeträgerabscheider 12, der einen Abzug zu einem Staubabscheider 13 (Abgasfilter oder Zyklon) besitzt, in dem Abgas B und der Staub C getrennt werden.

Wie die Vorrichtung gemäß Figur 1 betrieben wird, wird aus dem nachfolgenden Beispiel ersichtlich.

### Beispiel gemäß der Erfindung:

Über den Polymervorlagebehälter 1 werden pro Stunde 1000 kg PMMA-Abfallgranulat über Dosierschnecke 2 in den Reaktor 3 dosiert. Aus dem Vorlagebehälter 11 werden gleichzeitig 10.000 kg/h auf ca. 550 °C erhitzter Sand in den Reaktor 3 dosiert, in dem sich aufgrund der Mischung der o. g. Ströme eine Depolymerisationstemperatur von 400 °C einstellt.

Bei der Depolymerisation entstehen 5 kg/h feste Rückstände sowie 995 kg/h Gase und Dämpfe, welche über einen Zyklon 4 von Staub weitgehend befreit in den Kondensator 5 eingeleitet werden. Dort werden sie mit im Monomerkreislaufkühler 8 auf 25 °C abgekühlten Kondensat beaufschlagt und kondensiert, bevor das Kondensat in den Monomerbehälter 6 einfließt. Über eine Pumpe 7 wird der Monomerstrom durch den Kreislaufkühler 8 gepumpt und teils wieder zur Kondensation von Monomer-Dämpfen verwendet sowie in einer Menge von 990 kg/h zur Austragung A geführt. Die bei der Depolymerisation entstehenden 5 kg/h nicht kondensierbaren Gase werden über das Gebläse 16 abgesaugt und der Steigleitung 10 zugeführt, wo sie verbrannt werden.

Die 10 000 kg/h Sand aus dem Gaser 9 fließen mit 400 °C zusammen mit den 5 kg/h Rückständen aus der Depolymerisation in die Steigleitung 10 und werden durch das in der Brennkammer 15 gebildete Heißgas über die Steigleitung 10 pneumatisch in den Wärmeträgerabscheider 12 gefördert und auf 550 °C rückerhitzt. Am Fuß der Steigleitung 10 wird über Leitung 17 Zusatzbrennstoff, z. B. Heizöl, zugegeben. Durch den überschüssigen Luftsauerstoff aus der Brennkammer 15 werden der Zusatzbrennstoff sowie der organische Depolymerisations-Rückstand verbrannt bzw. oxidiert. Im Vorlagebehälter 11 trennen sich die anorganischen Pigmente und der Wärmeträgerabrieb vom grobkörnigeren Sand durch Sichtung ab. Dieser fällt in der Vorlage 11 an, während das Rauchgas und der Feinstaub in den Staubabscheider 13 gelangen, wo Gas B von Staub C getrennt wird.
Die Menge an aus dem Kondensat A gewonnenem MMA beträgt 958 kg/h (95,8 % Ausbeute).

### Bezugszeichenliste:

| Nr. | Bezeichnung |
|---|---|
| A | MMA-Produktstrom |
| B | Abgas |
| C | Staub |
| 1 | PMMA-Vorlagebehälter |
| 2 | Dosierschnecke |
| 3 | LR-Mischer-Reaktor |
| 4 | Zyklon |
| 5 | Kondensator |
| 6 | Monomerbehälter |
| 7 | Monomer-Kreislaufpumpe |
| 8 | Monomer-Kreislaufkühler |
| 9 | Gaser |
| 10 | Steigleitung |
| 11 | Wärmeträgersammelbehälter |
| 12 | Wärmeträgerabscheider |
| 13 | Staubabscheider |
| 14 | Brennerluftgebläse |
| 15 | Brennkammer |
| 16 | Abgasgebläse |
| 17 | Brennstoffleitung |

Der Reaktor 3 des Beispiels ist ein LR-Mischer-Reaktor, der an sich bekannt ist, z. B. aus "Erdoel und Kohle-Erdgas-Petrochemie/Hydrocarbon Technology" Nr. 42 (1989), Seiten 235 - 237. Der Reaktor arbeitet mit ineinandergreifenden, gleichsinnig rotierenden Wellen.

## Patentansprüche

1. Verfahren zur Rückgewinnung von monomeren Estern substituierter oder unsubstituierter Acrylsäure aus entsprechende Struktureinheiten aufweisendem Polymermaterial, bei dem man das Polymermaterial in einem Reaktor mit 400 °C bis 900 °C heißem mechanisch verwirbelten, feinkörnigen, festen Wärmeträger in Kontakt bringt,
**dadurch gekennzeichnet, dass** die dem Reaktor zugeführten Substanzen durch eine oder mehrere ineinandergreifende, rotierende Wellen, die mit Wendeln oder anderen Mischwerkzeugen versehen sind, mechanisch verwirbelt, daß man die Depolymerisation zwischen 300 °C und 650 °C durchführt, und die dabei entstehenden Dämpfe ableitet und kondensiert, wobei man den Wärmeträger im Reaktor durch die rotierenden Wellen, die mit Wendeln oder anderen Mischwerkzeugen versehen sind, von dessen Eintragsöffnung zur Austragsöffnung transportiert und den heißen Wärmeträger kontinuierlich an einem Ende des Reaktors zuführt und am anderen Ende abgekühlten Wärmeträger austrägt.

2. Verfahren nach Anspruch 1, **dadurch gekennzeichnet, daß** man den Wärmeträger nach dem Austrag aus dem Reaktor durch eine Aufheizung von Rückständen der Depolymerisation befreit.

3. Verfahren nach Anspruch 2, **dadurch gekennzeichnet, daß** man die Aufheizung des Wärmeträgers aus dem Reaktor mittels eines heißen Gasstromes, der ggf. sauerstoffhaltig sein kann, und/oder durch Zusatzbrennstoffe durchführt.

4. Verfahren nach Anspruch 3, **dadurch gekennzeichnet, daß** man den Gasstrom zum Befördern des Wärmeträgers in den Wärmeträgersammelbehälter (11) verwendet.

5. Verfahren nach einem der vorhergehenden Ansprüche, **dadurch gekennzeichnet, daß** der Wärmeträger eine Komgröße zwischen 0,1 und 5 mm besitzt.

6. Verfahren nach einem der vorhergehenden Ansprüche,
**dadurch gekennzeichnet, daß** man als Wärmeträger Oxide auf Basis von Silicium, Aluminium, Magnesium, Zirkonium oder Mischungen aus diesen Elementen verwendet.

7. Verfahren nach Anspruch 6, **dadurch gekennzeichnet, daß** man als Wärmeträger Sand verwendet.

8. Verfahren nach einem der vorhergehenden Ansprüche, **dadurch gekennzeichnet, daß** die Verweilzeit der im Reaktor gebildeten Dämpfe und Gase vor dem Kondensator weniger als 6 Sekunden, vorzugsweise weniger als 2 Sekunden, beträgt.

9. Verfahren nach einem der vorhergehenden Ansprüche **dadurch gekennzeichnet, daß** die Verweilzeit der feinkörnigen Wärmeträger-Feststoffe im Reaktor frei wählbar im Bereich von 5 bis 60 Sekunden liegt.

10. Verfahren nach einem der vorhergehenden Ansprüche, **dadurch gekennzeichnet, daß** Polymethylmethacrylat (PMMA) depolymerisiert wird und daß das Verhältnis von heißem Wärmeträger zu PMMA im Reaktor frei wählbar im Bereich von 3 : 1 bis 30 : 1 liegt.

11. Verfahren zur Rückgewinnung von monomeren Estern substituierter oder unsubstituierter Acrylsäure aus entsprechende Struktureinheiten aufweisendem Polymermaterial gemäß Anspruch 1, **dadurch gekennzeichnet, daß** man die abgeleiteten Dämpfe mit Kondensat, welches in einem Monomer-Kreislaufkühler abgekühlt wurde, behandelt und daß man das beim Behandeln resultierende Kondensat in den Monomer-Kreislaufkühler leitet, wo man es abkühlt und teilweise zum Behandeln der abgeleiteten Dämpfe rückführt, und man den übrigen Teil zur weiteren Aufarbeitung und Produktgewinnung ausschleust.

12. Verfahren nach Anspruch 11, **dadurch gekennzeichnet, daß** die abgeleiteten Dämpfe im Gleichstromverfahren mit Kondensat gequencht werden.

13. Verfahren nach Anspruch 11 und/oder 12, **dadurch gekennzeichnet, daß** das zum Behandeln der Monomergase verwendete Kondensat im Monomer-Kreislaufkühler auf eine Temperatur zwischen 5 und 40 °C, bevorzugt 20 und 30 °C, abgekühlt wird.

14. Verfahren nach einem oder mehreren der vorhergehenden Ansprüche, **dadurch gekennzeichnet, daß** die nicht kondensierbaren Gase und Dämpfe aus der Monomerkondensation in der Steigleitung verbrannt werden.

## Claims

1. A process of recovering monomeric esters of substituted or unsubstituted acrylic acid from polymer material having corresponding structural units, in which the polymer material is brought into contact with a hot, mechanically fluidised, fine-grained, solid heat-transfer medium at 400°C to 900°C in a reactor, **characterised in that** the substances supplied to the reactor are mechanically fluidised by one or more intermeshing, rotating shafts which are provided with helixes or other mixing implements, that the depolymerisation is performed between 300°C and 650°C, and the resulting vapours are withdrawn and condensed, the heat-transfer medium being transported in the reactor by the rotating shafts, which are provided with helixes or other mixing implements, from the inlet opening to the outlet opening and the hot heat-transfer medium being continuously supplied at one end of the reactor, and cooled heat-transfer medium being discharged at the other end.

2. A process according to Claim 1, **characterised in that** upon discharge from the reactor the heat-transfer medium is liberated from depolymerisation residues by means of heating.

3. A process according to Claim 2, **characterised in that** heating the heat-transfer medium from the reactor is effected by means of a hot gas stream, which may possibly be oxygen-containing, and/or by means of additional fuels.

4. A process according to Claim 3, **characterised in that** the gas stream is used for moving the heat-transfer medium into the heat-transfer medium receiver (11).

5. A process according to one of the preceding claims, **characterised in that** the heat-transfer medium has a grain size between 0.1 and 5 mm.

6. A process according to one of the preceding claims, **characterised in that** oxides based on silicon, aluminium, magnesium, zirconium or mixtures of these elements are used as heat-transfer medium.

7. A process according to Claim 6, **characterised in that** sand is used as heat-transfer medium.

8. A process according to one of the preceding claims, **characterised in that** the dwell time of the vapours and gases formed in the reactor before the condenser is less than 6 seconds, preferably less than 2 seconds.

9. A process according to one of the preceding claims, **characterised in that** the dwell time of the fine-grained heat-transfer solids in the reactor is freely selected in the range from 5 to 60 seconds.

10. A process according to one of the preceding claims, **characterised in that** polymethyl methacrylate (PMMA) is depolymerised and that the ratio of hot heat-transfer medium to PMMA in the reactor is freely selectable in the range from 3:1 to 30:1.

11. A process of recovering monomeric esters of substituted or unsubstituted acrylic acid from polymer material having corresponding structural units according to Claim 1, **characterised in that** the vapours withdrawn are treated with condensate which was cooled in a monomer circulating cooler, and that the condensate resulting from the treatment is introduced into the monomer circulating cooler, where it is cooled and in part recirculated for treating the vapours withdrawn, and the remaining part is discharged for further processing and product recovery.

12. A process according to Claim 11, **characterised in that** the vapours withdrawn are quenched with condensate in a co-current process.

13. A process according to Claim 11 and/or 12, **characterised in that** the condensate used for treating the monomer gases is cooled in the monomer circulating cooler to a temperature between 5 and 40°C, preferably 20 and 30°C.

14. A process according to one or more of the preceding claims, **characterised in that** the non-condensable gases and vapours from the monomer condensation are burnt in the riser.

## Revendications

1. Procédé de récupération d'esters monomères d'acide acrylique substitué ou non substitué à partir de matières polymères ayant des motifs de structure correspondants, dans lequel on met la matière polymère dans un réacteur en contact avec un agent caloporteur solide en grains fins mis en tourbillonnement mécaniquement et ayant une température de 400°C à 900°C,
**caractérisé en ce que** l'on met en tourbillonnement mécaniquement les substances envoyées au réacteur par un ou par plusieurs arbres tournants qui s'interpénètrent et qui sont munis d'hélices ou d'autres outils de mélange, **en ce que** l'on effectue la dépolymérisation entre 300°C et 650°C, et on évacue les vapeurs qui se forment et on les condense, on transporte l'agent caloporteur dans le réacteur par les arbres tournants qui sont munis d'hélices ou d'autres outils de mélange, de l'ouverture d'entrée du réacteur à l'ouverture de sortie, et on envoie l'agent caloporteur chaud en continu à une extrémité du réacteur tandis que l'on soutire à l'autre extrémité de l'agent caloporteur refroidi.

2. Procédé suivant la revendication 1, **caractérisé en ce que** l'on débarrasse de résidus de dépolymérisation l'agent caloporteur après la sortie du réacteur par un chauffage.

3. Procédé suivant la revendication 2, **caractérisé en ce que** l'on effectue le chauffage de l'agent caloporteur qui sort du réacteur au moyen d'un courant gazeux chaud qui peut, le cas échéant, contenir de l'oxygène et/ou par des combustibles supplémentaires.

4. Procédé suivant la revendication 3, **caractérisé en ce qu'**on utilise le courant gazeux pour transporter l'agent caloporteur dans la cuve (11) collectrice d'agent caloporteur.

5. Procédé suivant l'une des revendications précédentes, **caractérisé en ce que** l'agent caloporteur a une granulométrie comprise entre 0,1 et 5 mm.

6. Procédé suivant l'une des revendications précédentes, **caractérisé en ce que** l'on utilise comme agent caloporteur des oxydes à base de silicium, d'aluminium, de magnésium, de zirconium ou de mélange de ces éléments.

7. Procédé suivant la revendication 6, **caractérisé en ce que** l'on utilise du sable comme agent caloporteur.

8. Procédé suivant l'une des revendications précédentes, **caractérisé en ce que** la durée de séjour des vapeurs et des gaz formés dans le réacteur avant le condensateur est inférieure à 6 secondes et, de préférence, est inférieure à 2 secondes.

9. Procédé suivant l'une des revendications précédentes, **caractérisé en ce que** la durée de séjour des matières solides formant caloporteur à grains fins dans le réacteur peut être choisie librement entre 5 et 60 secondes.

10. Procédé suivant l'une des revendications précédentes, **caractérisé en ce que** l'on dépolymérise le poly(méthacrylate de méthyle) (PMMA) et que le rapport du caloporteur chaud au PMMA dans le réacteur peut être choisi librement entre 3:1 et 30:1.

11. Procédé de récupération d'esters monomères d'acide acrylique substitué ou non substitué à partir d'une matière polymère ayant des motifs de structure correspondants suivant la revendication 1, **caractérisé en ce que** l'on traite les vapeurs évacuées par du produit condensé qui a été refroidi dans un dispositif de refroidissement en circuit fermé du monomère, et **en ce que** l'on envoie le produit condensé résultant du traitement dans le dispositif de refroidissement en circuit fermé du monomère où on le refroidit et on le retourne en partie au traitement des vapeurs évacuées, et on évacue la partie restante vers un autre traitement et une récupération du produit.

12. Procédé suivant la revendication 11, **caractérisé en ce que** l'on refroidit brusquement par du produit condensé les vapeurs évacuées suivant un procédé en courant de même sens.

13. Procédé suivant la revendication 11 et/ou 12, **caractérisé en ce que** l'on refroidit à une température comprise entre 5 et 40°C, et de préférence entre 20 et 30°C, le produit condensé utilisé pour le traitement des gaz monomères dans le dispositif de refroidissement en circuit fermé du monomère.

14. Procédé suivant l'une ou plusieurs des revendications précédentes, **caractérisé en ce que** l'on brûle dans le conduit ascendant les gaz et les vapeurs non condensables provenant de la condensation du monomère.
